(19)

Europäisches
Patentamt
European
Patent Office
Office européen
des brevets

(11)     **EP 2 086 314 B1**

(12)                    **EUROPEAN PATENT SPECIFICATION**

(45) Date of publication and mention
of the grant of the patent:
**08.08.2012 Bulletin 2012/32**

(51) Int Cl.:
**B05B 17/06** (2006.01)      **A01M 1/20** (2006.01)
**A61L 9/14** (2006.01)

(21) Application number: **07838698.4**

(22) Date of filing: **21.09.2007**

(86) International application number:
**PCT/US2007/020546**

(87) International publication number:
**WO 2008/039393 (03.04.2008 Gazette 2008/14)**

(54) **METHOD OF DISTRIBUTING A LIQUID ACTIVE MATERIALS USING AN ULTRASONIC TRANSDUCER**

VERFAHREN ZUR ABGABE AKTIVER FLÜSSIGMATERIALIEN MITTELS ULTRASCHALLWANDLER

METHODE DE DISTRIBUTION DE MATIÈRES LIQUIDES ACTIVES UTILISANT UN TRANSDUCTEUR ÉLECTROMÉCANIQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **22.09.2006  US 846598 P**

(43) Date of publication of application:
**12.08.2009 Bulletin 2009/33**

(73) Proprietor: **The Procter & Gamble Company Cincinnati, OH 45202 (US)**

(72) Inventors:
• **TOLLENS, Fernando Ray**
**Cincinnati Ohio 45140 (US)**
• **DIERSING, Steven Louis**
**Cincinnati Ohio 45211 (US)**

• **HECHT, John Philip**
**West Chester Ohio 45069 (US)**
• **SCHROECK, Steven James**
**Cincinnati Ohio 45241 (US)**
• **MAHONEY, William Paul III**
**Liberty Township Ohio 45011 (US)**

(74) Representative: **Morelle, Evelyne Charlotte Isabelle**
**N.V. Procter & Gamble Services Company S.A.**
**Temselaan 100**
**1853 Strombeek-Bever (BE)**

(56) References cited:
**WO-A-03/047766      WO-A-03/068413**
**US-A1- 2006 175 426      US-B1- 6 293 474**

**Description**

FIELD OF THE INVENTION

The present invention relates to a method of distributing liquid active materials, such as perfumes, air fresheners, insecticide formulations, and volatile materials, to the atmosphere by means of an electromechanical transducer.

BACKGROUND OF THE INVENTION

A number of processes exist for the generation of liquid droplets using electromechanical actuation. One method for such distribution is to atomize a liquid by a delivery system comprising a perforate structure which is vibrated by an electromechanical transducer which has a composite thin-walled or planar structure, and is arranged to operate in a bending mode. Liquid is supplied to the vibrating perforate structure and sprayed therefrom in droplets upon vibration of the perforate structure. Such attempts in the art are illustrated in U.S. Pat. Nos. 3,543,122, 3,613,041, 4,479,609, 4,533,082, 4,790,479, 5,519,179, 5,297,734, 6,341,732, 6,378,780, and 6,396,462 and in documents WO 03/047766 and US 2006/0175 426.
While electromechanical transducer delivery systems are known in art, their difficulties are known as well. Specifically, there have been efforts to improve the overall distribution of the atomized liquids in a volume of space. However, many of these delivery systems suffer because of their inability to properly "loft" the atomized liquids in the air high enough to achieve sufficient dispersion such that the distribution of the liquid active materials is maximized. Thus, a need exists for an improved delivery system .and method for generating droplets of liquid active materials to increase the distribution of the liquid active materials beyond that which is currently capable.

SUMMARY OF THE INVENTION

The invention relates to a method of distributing liquid active materials to an atmosphere comprising the steps of: providing a quantity of the liquid active materials; providing an electromechanical transducer in communication with the liquid active material; activating the electromechanical transducer to distribute the liquid active material to the atmosphere; and carrying out the activation for a first period of time of between 60 and 500 milliseconds wherein said electromechanical transducer is coupled or decoupled to a perforated structure having a plurality of perforations (2a, 2b, 2c), said perforations having a diameter of 4 to 7 microns.

BREF DESCRIPTION OF THE DRAWINGS

While the specification concludes with the claims particularly pointing and distinctly claiming the invention, it is believed that the present invention will be better understood from the following description taken in conjunction with the accompanying drawings in which:

Fig. 1 is a perspective view of one embodiment of a delivery system of the present invention;
Fig.2 is across-sectional view of one embodiment of a delivery system of the present invention;
Fig. 3 is an enlarged top view of one embodiment of the droplet generation element of the present invention;
Fig. 4 is an enlarged cross-sectional view of the droplet generation element in Fig. 3.

DETAILED DESCRIPTION OF THE INVENTION

The present invention relates to the method described above. Without wishing to be bound by theory, it is believed that controlling the length of the first period and the second period provides a surprising increase in the distribution of the volatile materials contained within the liquid active materials and reduction of the hedonic habituation of the user. By controlling the length of the first period of time and the second period of time, the transition or evaporation of liquid droplets to finer droplets and/or a gaseous state as a means to increase liquid active material distribution is improved. The length of the first period controls the increased evaporation rate of the droplets of the liquid active materials, enhancing their transition to an atomized or molecular state and increasing their ability to remain airbone, disperse, and diffuse. In turn, this increases the room fill capability of the liquid active materials. The second period or the deactivation period, in one aspect, is used to reduce the level of habituation by the user. Habituation is a process used by the olfactory sensory system to reduce its sensitivity to a previously detected scent. Without wishing to be bound by theory, the second period of time allows for the volatile concentration of the liquid active materials to reduce below the habituation level threshold allowing the reset of the olfactory system and the re-detection or re-perception of the scent. In effect, the first and second periods surprisingly increase the perceived intensity of the liquid active formulation.

Timing Mechanism

Controlling the length of the first period and second period according to the present invention creates chimney or "airbus" effect wherein the amount of air entrainment as well as the velocity of the droplets increase, resulting in an increased concentration of airborne volatile materials form the liquid active materials as evidence by the higher detection of components. This effect improves the perceived intensity and hedonics of the liquid active material. The timing mechanism can be controlled by any of the known intelligent systems in the art. The first period and second period, together forming an operating cycle, can be repeated at least once. In another embodiment, the operating cycle is repeated until the liquid active material is exhausted and/or the power supply is exhausted.

In one embodiment which is not part of the invention, the first period is at least about 5 milliseconds; in another embodiment which is not part of the invention at least about 10 milliseconds; in another embodiment which is not part of the invention at least about 20 milliseconds; in another embodiment which is not part of the invention at least about 50 milliseconds; in another embodiment which is not part of the invention at least about 100 milliseconds; in another embodiment which is not part of the invention at least about 250 milliseconds; in another embodiment which is not part of the invention at least about 500 milliseconds; in another embodiment which is not part of the invention at least about 1 second; in another embodiment which is not part of the invention at least about 5 seconds. In another embodiment which is not part of the invention, the first period is between about 5 milliseconds and about 5 seconds; in another embodiment which is not part of the invention about 10 milliseconds and 1 second; in another embodiment which is not part of the invention between about 20 milliseconds and 500 milliseconds; in another embodiment which is not part of the invention between about 50 milliseconds and 100 milliseconds.

In another embodiment which is not part of the invention, the delivery system for generating droplets of liquid active material comprises; a reservoir wherein the reservoir contains a liquid active material; an electromechanical transducer, upon excitation by a power supply; a droplet generation element operatively associated with said electromechanical transducer and positioned for contact with said liquid active material; a timing mechanism for activating said electromechanical transducer over a first period, and alternatingly deactivating said electromechanical transducer over a second period; and wherein said timing mechanism activates said electromechanical transducer for a first period is between 20 and 120 milliseconds. Alternatively, the first period is between 25 and 120 milliseconds, alternatively between 50 and 120 milliseconds, alternatively between 50 and 100 milliseconds and alternatively between 60 and 80 milliseconds.

In one embodiment, the second period is between about 1 second and 30 hours; in another embodiment between about 1 second and 24 hours; in another embodiment between about 5 seconds and 12 hours; in another embodiment between about 5 seconds and 8 hours; in another embodiment between about 5 seconds and 6 hours; in another embodiment between about 5 seconds and 4 hours; in another embodiment between about 5 seconds and 3 hours; in another embodiment between about 5 seconds and about 2 hours; in another embodiment between about 5 seconds and about 1 hour; in another embodiment between about 5 seconds and 45 minutes; in another embodiment between about 5 seconds and 30 minutes; in another embodiment between about 5 seconds and 20 minutes; in another embodiment between about 5 seconds and 15 minutes; in another embodiment between about 5 seconds and 10 minutes; in another embodiment between about 5 seconds and about 5 minutes; in another embodiment between about 5 seconds and about 150 seconds; in another embodiment between about 20 seconds and about 120 seconds; in another embodiment between about 25 seconds and about 80 seconds; in another embodiment between about 10 seconds and about 40 seconds. It is understood, however, that periods of time longer than the above ranges of time may be utilized with the present invention.

Liquid Active Materials

The liquid active materials of the present invention include volatile materials, nonvolatile materials, and combinations thereof. The liquid active materials of the present invention readily flow at temperatures ofbetween about 10°C and about 30°C. The liquid active materials maybe generated in various facilities, which include but are not limited to rooms, houses, hospitals, offices, theaters, buildings, and the like, or into various vehicles such as trains, subways, automobiles, airplanes, the outdoors and the like. The volatile materials of interest herein can be in any suitable form including, but not limited to: dispersion of solids, emulsions, liquids, and combinations thereof. For example, the delivery system may contain a volatile material comprising a single-phase composition, multiphase composition and combinations thereof, from one or more sources in one or more carrier materials (e.g. water, solvent, etc.).

Nonvolatile materials, including solids, are also contemplated for use with the present invention. It is believed that when nonvolatile materials are part of the liquid active material, the nonvolatile materials are finely dispersed in the air into particles capable of being at least partially carried by air.

It should be understood that when the droplets of liquid active materials are described herein as being "distributed", "generated" or "released", this refers to the volatilization of the evaporative components of the volatile materials and to the release to the atmosphere of the non-evaporative components, which may be small solids or particulates.

The term "solids" as used herein, refers to a material that has a tangible or concrete form as discrete material at room temperature (22 °C), that is, they tend to keep their form rather than flow or spread out like liquids or gases. The solids may be dissolved in the formulation or suspended throughout. Solids may behave very similar to base notes as they bring depth and body to a perfume.

The terms "volatile materials", "aroma", and "scents", as used herein, include, but are not limited to pleasant or savory smells, and, thus, also encompass scents that function as fragrances, deodorizers, odor eliminators, malodor counteractants, insecticides, insect repellants, medicinal substances, air fresheners, deodorants, aromacology, aromatherapy, or any other odor that acts to condition, modify, or otherwise charge the atmosphere or to modify the atmosphere.

In addition, the term "volatile materials" as used herein, refers to a material or a discrete unit comprised of one or more materials that is vaporizable, or comprises a material that is vaporizable without the need of an energy source. Any suitable volatile material in any amount or form may be used. The term "volatile materials" includes but is not limited to compositions that are comprised entirely of a single volatile material. It should be understood that the term "volatile material" also refers to compositions that have more than one volatile component, and it is not necessary for all of the component materials of the volatile material to be volatile. The volatile materials described herein may, thus, also have non-volatile components.

The volatile material may comprise a perfume, although the invention is not so limited. A perfume may include a single aromatic chemical or a mixture of aromatic chemicals. As used herein, aromatic chemicals mean chemicals that have an odor. There are several chemical classes which fall within aromatic chemicals, including but not limited to ionones, hydrocarbons, alcohols, aldehydes, ketones, esters, etc.

The term "fragrance" or "perfume" refers to all organic substances which have a desired olfactory property and are essentially nontoxic. They can be compounds of natural, semi synthetic or synthetic origin. A fragrance can be a combination of various odorous substances which evaporate at different rates and/or during different periods. Fragrance can exhibit what is known as a "top note," which may be the odor which first diffuses when the fragrance is applied, generated or released to the atmosphere, a "heart note" or "middle note," which may complete or complement the fragrance providing body and texture, and a "base note," which may be the most substantive odor and can be perceived several hours after application or emission.

In order to be noticeable, a perfume has to be volatile, its molecular weight being an important factor along with the nature of the functional groups and the structure of the chemical compound. Thus, most perfumes have molecular weights of up to about 200 Dalton, with molecular weights of 300 Dalton and higher being more the exception. In view of the differences in volatility of perfumes, the odor of a perfume or fragrance composed of several perfumes changes during the evaporation process, the odor impressions being divided into the top note, the middle note or body and the base note.

Since odor perception is also based to a large extent on odor intensity, the top note of a perfume or fragrance may not consist solely of readily volatile compounds. The base note may consist largely of less volatile, i.e. firmly adhering, perfumes. In the composition of perfumes, more readily volatile perfumes may be fixed, for example, to certain "fixatives", which prevents them from vaporizing too rapidly. The perfume may also contain small amounts of other additives, such as solvents, preservatives, antioxidants, UV screening agents and the like. The fragrance matrix may also include organoleptic components, such as for example, other well-known fragrance ingredients.

The fragrances or perfumes may include natural and/or synthetic oils, extracts and/or essences which may comprise complex mixtures of constituents, such as orange oil, lemon oil, rose extract, lavender, musk, patchouli, balsam essence, sandalwood oil, pine oil, and cedar oil.

A useful term to quantify the degree of volatility of the volatile materials is the Kovat's Index. The Kovat's Index (KI, or Retention Index) may be defmed by he selective retention of solutes or perfume raw materials (PRMs) onto the chromatographic columns. It is primarily determined by the column stationary phase and the properties of solutes or PRMs. For a given column system, a PRM's polarity, molecular weight, vapor pressure, boiling point and the stationary phase property determine the extent of retention. To systematically express the retention of analyze on a given GC column, a measure called Kovat's Index is defined. The Kovat's Index places the volatility attributes of an analyte (or PRM) on a column in relation to the volatility characteristics of n-alkane series on that column. Typical columns used are DB-5 and DB-1.

By this definition the KI of a normal alkane may be set to 100n, where n = number of C atoms of the n-alkane. With this definition, the Kovat's index of a PRM, x, eluting at time t', between two n-alkanes with number of carbon atoms n and N having corrected retention times t'n and t'N respectively will then be calculated as:

$$KI = 100x(n + \frac{\log t'_x - \log t'_n}{\log t'_N - \log t'_n})(1)$$

This equation can be used to calculate the Kovat's index for any volatile material. Furthermore, this equation can be used to further separate volatile components into three categories; top, middle and base notes. Using the Kovat's index, a top note may as have a KI less than or equal to 1200, a middle note between 1200 and 1400, and a base note greater than or equal to 1400. For example, a typical perfume formulation having 2 percent solids may comprise 70 percent top notes, 20 percent middle notes, and 10 percent bottom notes. A comparable formulation having about 3 percent solids may comprise about 40 to about 60 percent, particularly about 50 percent top notes; about 20 to about 40 percent, particularly about 30 percent middle notes; and about 10 to about 30 percent, particularly about 20 percent bottom notes.

Delivery System

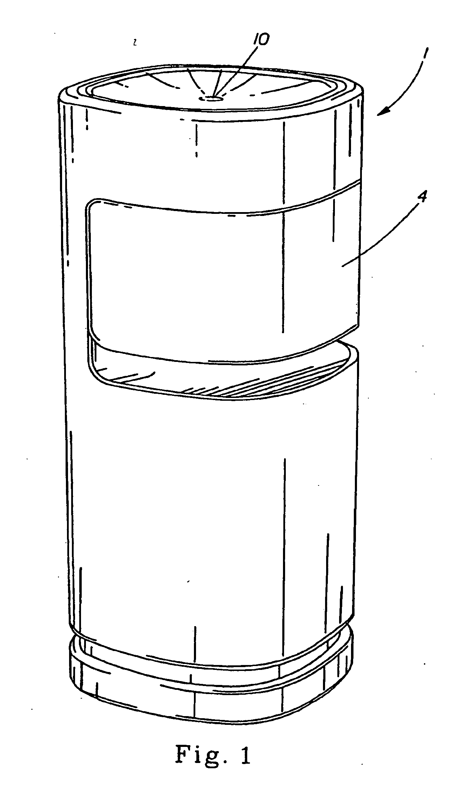

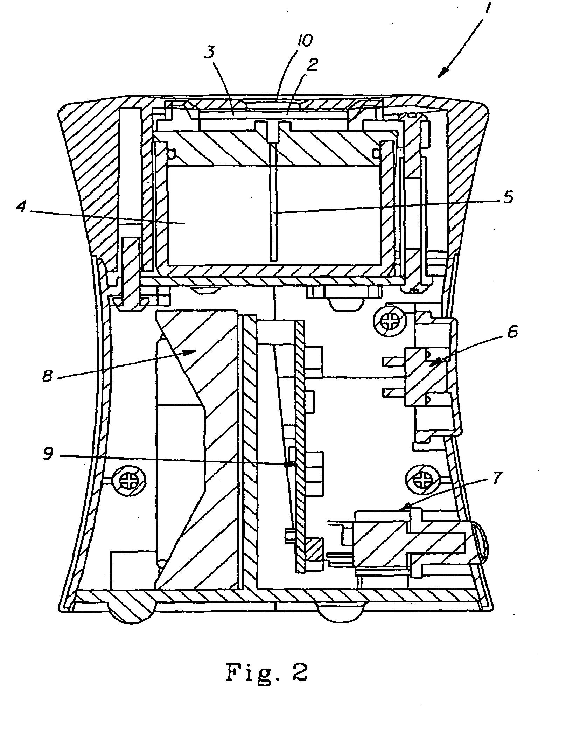

Now referring to Figures 1 and 2, a non-limiting and exemplary delivery system 1 is shown. The delivery system 1 includes a liquid reservoir 4, which contains a liquid active material to be atomized, that may be juxtaposed with and mounted below the electromechanical transducer 3 and droplet generation element 2. The liquid supply component 5 may extend upwardly from within the reservoir 4 to the rear face of the droplet generation element 2 or to a region in fluid communication with the droplet generation element 2. Upon activation of the electromechanical transducer 3, the liquid active material is generated through the droplet generation element 2, the orifice 10, and into the atmosphere.

The reservoir 4 may comprise any liquid tight container suitable for holding an adequate quantity of the liquid active materials to be dispensed. The reservoir 4 may be pressurized to provide for delivery of the liquid active materials to the droplet generation element 2, or may be maintained at atmospheric pressure. Upon depletion of the reservoir 4, the reservoir 4 may be refillable with liquid active material provided from a bulk supply or the reservoir 4 may be replaced with a new reservoir containing a quantity of liquid active material.

The liquid active materials may be delivered to a droplet generation element 2 by a liquid supply component 5 working by gravity feed, capillary action, pumping action, etc. When the droplet generation element 2 is a perforate structure, the liquid supply component 5 may be disposed near the center of the droplet generation element 2 so that the liquid supply component 5 may contact the perforations 2a, 2b, 2c of the droplet generation element 8. However, the liquid supply component 5 need not contact the perforations 2a, 2b, 2c and the perforations 2a, 2b, 2c may be laterally displaced from the liquid supply component 5.

A continuous feed of the liquid active material from the reservoir 4 to the droplet generation element 2 may be desired. The continuous feed may be accomplished by a using liquid supply component 5, which may comprise a feed tube that delivers liquid active material to the rear face of the droplet generation element 2 or to a position juxtaposed with the rear face of the droplet generation element 2. The rear face of the droplet generation element 2 is the face opposite from which the droplets emerge. The liquid active materials may be delivered from the reservoir 4 to one face of the droplet generation element 2 by a capillary feed. The capillary feed may be flexible and have a surface or assembly of surfaces over which liquid active material can pass from the reservoir 4 towards the droplet generation element 2. Exemplary capillary material forms include open cell foams, fibrous wicks, porous plastic wicks, and glass or polymeric capillary tubes.

In applications where relatively high droplet production rates and/or a relatively high percentage of solid are desired, capillary feed may be provided by a relatively open structure, which may also be relatively rigid. This arrangement may provide the advantage of a relatively large, unrestricted area for liquid flow for a given surface area at the wall of the capillary tube. In such a liquid transfer process the area between the capillary material surfaces through which liquid may flow to the capillary surfaces, i.e., liquid volume is relatively large to the surface area of the capillary surfaces. This geometry may provide a liquid transfer process which is less restrictive than a similar transfer process utilizing a porous capillary wick. Without being bound by theory, it is believed that an open tube capillary may minimize the interaction between the capillary system porous media and the dispersed solids, thereby allowing the solids to be generated with the droplets as part of the bulk liquid with minimal liquid to solid separation. The open capillary tube may have a delivery rate of at least about 20, about 30 or about 40 mg/hr, but is typically not more than about 80, about 70, about 60 or about 50 mg/hr.

The capillary tube may have a closed cross section, such as a circle. Alternatively, the closed cross section may be non-circular, such as an oval, square, etc. Alternatively, the open capillary tube may comprise an open channel, such as a weir, etc. The capillary tube may be rigid and made of glass, polymeric materials, etc.

Furthermore, in applications where the droplet production rates in the range of 1 mg/s or more are desired, flat channel capillaries may offer the benefit of a relatively greater delivery rate with a simple design and ease of manufacturing. When using a flat channel design consideration may be given to the height of the capillary, since the drag pull on a flat capillary channel is half of that of a capillary tube, resulting in only half of the capillary rise compared to a closed tube.

If desired, plural capillary tubes may be used in parallel to transport liquid from the reservoir to the actuator. If plural capillary tubes are used, the capillary tubes may be of equal or unequal length, cross-sectional area, cross-sectional shape, length, delivery rate, etc. The capillary tubes may have a common or different origin within the reservoir. Alternatively, plural capillary tubes may be utilized to deliver a like number of plural liquids from separate reservoirs to a common perforate structure. This arrangement provides the advantage that incompatible materials may be kept apart, in discrete reservoirs, until these materials are dispensed at the point of use. The plural materials may be fed from their respective reservoirs to the perforate structure at the same flow rate or at different flow rates.

In yet another alternative embodiment having plural reservoirs, plural transducers and a like number of plural perforate structures may be utilized and operated in parallel. This arrangement provides the advantage that no mixing of separate materials occurs until the materials are dispensed into the atmosphere. Again, the materials may be dispensed at a common flow rate or at different flow rates. If so the plural reservoirs, transducers, perforate plates, etc. may be the same or may differ in function and/or performance.

The delivery system 1 comprises an electromechanical transducer 3, which is an element capable of converting electrical energy to mechanical energy. One known example of an electromechanical transducer 3 comprises piezoelectric materials, which have the ability to change shape when subject to an externally applied voltage. The voltage may cause the electromechanical transducer 3 to vibrate at certain frequencies. The electromechanical transducer 3 may be driven with an oscillating voltage at one of the resonant frequencies of the system or alternatively with a waveform that gives droplet on demand operation. The oscillating voltage may produce a vibration in the transducer. The vibration may, in turn, generates droplets of liquid active material through a droplet generation element 2, such as a perforate structure that is operatively associated with the electromechanical transducer 3. The droplets of liquid active material are then distributed to the atmosphere. It is believed that a resultant pressure differential may be induced in the liquid directly behind a perforate structure. The resulting pressure differential may force the liquid through the perforations of a perforate structure to form droplets.

The electromechanical transducer 3 may comprise a piezoelectric material, which vibrates at a resonant frequency under an externally applied voltage. The electromechanical transducer 3 may comprise various shapes and forms, such as a round disc. A disc-shaped transducer may have two opposed faces. A separate electrode may be disposed on each face and be radially poled. The electrodes may excite the length modes of the disc shaped transducer or a mode of a perforate structure.

The electrodes may be patterned so as to incorporate "drive" and "sense" electrodes. The drive and sense electrodes are electrically insulated but mechanically coupled through the piezoelectric transducer. A drive voltage may be applied to the drive electrode. The resulting motion in the transducer generates a voltage at the sense electrode. This voltage can then be monitored and used to control the drive voltage through a feedback circuit. The electrical response may be used to adjust the voltage to achieve specified resonances either by phase locking, amplitude maximizing or other known means. In order to maximize the electromechanical coupling to the desired mode it may be useful to shape the drive electrode appropriately.

The induced vibration may have an amplitude and phase induced in relation to characteristics of the drive signal. If desired, the drive voltage may sweep various frequencies, to provide a range of dispensing characteristics. Alternatively, the drive voltage may excite the transducer at a single frequency. The single frequency may be coincident or near the transducer's natural frequency or a harmonic thereof. This arrangement may provide the benefit that less power is consumed than using a sweep of multiple frequencies over a spectrum.

In may also be useful to incorporate a sense electrode into the delivery system 1. A sense electrode can give phase and amplitude information that allows an appropriate electronic circuit to lock on to the correct resonant mode. It may be advantageous to shape the sense electrode so as to achieve appropriate electromechanical coupling.

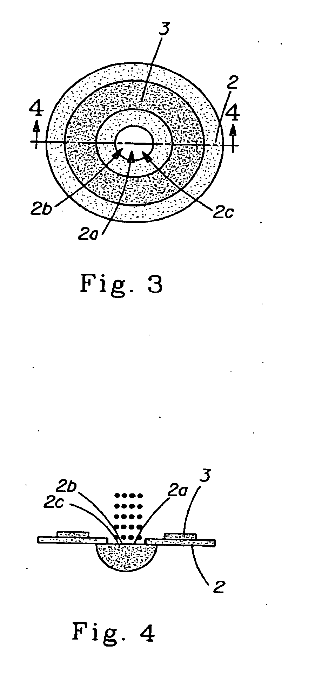

One embodiment of the electromechanical transducer 3 is shown in Figures 3 and 4. In this embodiment, the electromechanical transducer 3 can have a diameter of about 10 to about 50 millimeters. In certain embodiments the diameter may be less than about 25 millimeters, less than about 20 millimeters, or may be 15 S about millimeters or less. The electromechanical transducer 3 may have a centrally located aperture therethrough. The aperture may have a diameter of about 5 millimeters to about 15 millimeters. The electromechanical transducer 3 may be flat and vibrate in a bending mode, expanding mode or combination thereof with a major excursion generally perpendicular to the opposed faces of the transducer. The beading may be bilateral or unilateral. A suitable piezoelectric transducer is disclosed in U.S. Patent No. 5,518,179.

The droplet generation element 2 may be formed from a variety of materials including electro formed nickel, etched silicon, stainless steel or plastics. The droplet generation element 2 may be flexible or stiff. A flexible design is one where the amplitudes of the vibrational modes of the droplet generation element 2 are large compared with those of the electromechanical transducer 3. The resulting motion may have a significant effect on the droplet generation process. A stiff design is one where the amplitudes of the vibrational modes of the perforate structure are generally equal to or smaller than those of the electromechanical transducer. This motion generally follows the motion of the electromechanical transducer. In either design, the flexibility may be controlled by a choice of material and thickness. The benefit of the stiff design is that a stiff droplet generation element may generate uniform droplets across its surface without dampening the overall motion of the droplet generation element.

The droplet generation element 2 is operatively associated with the electromechanical transducer 3. "operatively associated" means that the droplet generation element is responsive to the activation of the electromechanical transducer 3 such that the liquid active material passes through the droplet generation element 2 for diffusion into the atmosphere. In one embodiment, the droplet generation element 2 is operatively associated with the electromechanical transducer 3 by being joined or coupled to one face of the electromechanical transducer 3 by adhesive, solder, etc. A non-limiting, exemplary coupled electromechanical transducer 3 is shown in Figure 4. The droplet generation element 2 may also be de-coupled from the electromechanical transducer 3, yet still be operatively associated with the transducer 3. A suitable de-coupled electromechanical transducer is described in WO 02/068128.

The droplet generation element 2 is a perforate structure, as shown in Figures 3 and 4, that comprises plural perforations 2a, 2b, 2c disposed on a pattern, such as a hexagonal lattice. It has been found that structuring the droplet generation element to maximize the flow rate of the liquid active material while minimizing deposition enhances the perceived intensity of the liquid active formulation.

The droplet size may be determined by varying the cross sectional area of the exit of the perforations 2a, 2b, 2c. Round perforations 2a, 2b, 2c have a diameter of about 4 to about 7 microns. In one embodiment which is not part of the invention, the diameter of the perforations 2a, 2b, 2c may be less than about 30 microns. In another embodiment which is not part of the invention, the diameter of the perforations 2a, 2b, 2c may be less than about 15 microns.

The perforations 2a, 2b, 2c may be tapered to have a reduction in cross-sectional area in the flow direction. If a perforate structure having perforations 2a, 2b, 2c of variable cross section is selected, the cross sectional area of the perforations 2a, 2b, 2c may decrease from the rear face to the front face of the perforate structure. Such a tapered perforation may reduce the amplitude of vibration of the perforate structure which is necessary in order to produce droplets of a given size, due to the reduction of viscous drag upon the liquid as it passes through such perforations 2a, 2b, 2c. Consequently, a relatively lower excitation of the electromechanical transducer 3 may be used, thereby providing improved efficiency in creating the droplets to be dispensed. In the case of a coupled electromechanical transducer and perforate structure, the relatively lesser excitation may enable the use of a relatively thick and robust perforate structure from which satisfactory droplet production can be achieved. This may also provide the successful creation of droplets from liquids of relatively high viscosity and may reduce the mechanical stresses in the perforate structure. In the case of a decoupled electromechanical transducer and perforate structure, the reduction of viscous drag will also result in improved efficiency with respect to the power that is necessary to generate the droplets.

The droplet generation element 2 may also be a non-perforate structure. For example, the liquid active material is fed onto a face of the droplet generation element 2 that is opposite the rear face. Droplets are generated by vibration of the droplet generation element 2 whether it is bending, expanding, bilateral or unilateral.

The delivery system 1 may have a first, disposable part, comprising the liquid and its container or liquid reservoir 4. The second part, may be reusable, and may comprise the electromechanical transducer 3, the droplet generation element 2 with its associated drive electronics 9 and a power supply 8. This provides a system which is refillable. Alternatively, the system may be discarded upon depletion of the reservoir.

The delivery system can be operated from any suitable power supply 8. The power supply 8 may be a battery, electrical power from a wall outlet, solar photovoltaic conversion, etc.

The delivery system may further comprise an automatic switch, as is known in the art. The automatic switch may activate or deactivate the delivery system when a threshold amount of energy is or is not present For example, the automatic switch may comprise a photocell. The photocell may cause the delivery system to shut down, when a threshold amount of light is not present. This allows the delivery system to shut down at night, in case people are not present during the evening. The photocell may shut down the either the fan, electromechanical transducer, or both. Alternatively, the delivery system, transducer and/or fan may be activated by, or be rendered inactive by, the presence or absence of sound, motion, heat or other energy forms.

The delivery system 1 may further comprise an intensity controller 9 for controlling the amount of liquid active material that is dispensed into the atmosphere. The delivery system 1 may also include a boost controller for providing bursts of liquid active material beyond the amount normally dispensed from the delivery system 1. In another embodiment a heater may be utilized to assist in and accelerate the volatilization of the liquid active materials.

The delivery system 1 may further comprise a light source. Suitable light sources include but are not limited to light generating diodes ("LEDs"), incandescent sources of light including but not limited to filament-based bulbs, and luminescent sources of light including but not limited to electroluminescent, chemiluminescent, cathodoluminescent, tribolumimescent, and photoluminscent materials. In one non-limiting embodiment the light source is one or more LEDs. The LED can be any number of colors including but not limited to yellow, white, red, green, blue, pink, or a combination thereof. One non-limiting example of an LED suitable for use with the present invention is part No. MV8305 (available from Fairchild Semiconductor of South Portland, Maine).

In one non-limiting embodiment, the light is positioned on the delivery system 1 such the light is directed towards the reservoir 4. It may be designed such that the light turns on automatically when the delivery system 1 is turned on or designed such that the light is controlled separately from the operation of the delivery system 1. If desired, the light source could be connected to a timer incorporated in the delivery system 1 such that the light automatically turns-off after a predetermined time period. Also, if desired, the light source may provide a light that is varying in intensity. In a further embodiment, the method of distributing a liquid active material into the atmosphere further comprises providing a quantity of a second liquid; (a) providing an electromechanical transducer (3) in communication with said second liquid; (b) activating said electromechanical transducer (3) to distribute said second liquid to said atmosphere; and carrying out said activation for an alternate period of time of at least 20 milliseconds. In an embodiment said liquid active material is distributed to said atmosphere for a first period of time and said second liquid is distributed to said atmosphere for said alternate period, said first period and said alternate period being different.

The following examples are presented for illustrative purposes, and are not intended, in any way, to limit the scope of the invention.

Example 1

Example 1 compares the effect of the length of activation period on perceived intensify using a piezoelectric delivery system according to the following Sensory Evaluation Method for delivery systems or apparatus.

A dedicated odor evaluation room is utilized for all sensory evaluations. A trained odor evaluator verifies that there is not any residual perfume or room odor present in the room. The door(s) to the room are closed and the delivery system or apparatus is activated by a test facilitator. Trained odor evaluators enter the odor evaluation room and perform odor evaluations at the following time intervals: (1) 3 minutes after activation (2) 6 minutes after activation (3) 12 minutes after activation and (4) 18 minutes after activation. The sensory evaluations are conducted at the following distances from the delivery system or apparatus starting at the furthest distance: (1) 0.9 meters (2) 1.8 meters and (3) 2.7 meters. Expert evaluators exit the room between odor evaluations and the door(s) are closed between odor evaluations. Expert evaluators provide odor intensity measurements on a sensory rating scale of 0-5.

Perfume Intensity Scale:

5 = very strong, i.e., extremely overpowering, permeates into nose, can almost taste it
4 = strong, i.e., very room filling, but slightly overpowering
3 = moderate, i.e., room filling, odor character clearly recognizable
2 = light, i.e., fills part of the room, with recognizable odor character
1 = weak, i.e., diffusion is limited, odor character difficult to describe,
0 = no scent

Table 1 illustrates the improved perfume hedonic data at all distances from the delivery system when the length of the activation time of the electromechanical transducer is increased at constant delivering rate. This translates to the consumer as better perfume intensity and character.

| Table 1 | Perfume intensity grade at given distance from the delivery system | | |
|---|---|---|---|
| | 0.9 meters | 1.8 meters | 2.7 meters |
| Short Pulse (10 ms) | 1.5 | 0.5 | 0.5 |
| Medium Pulse (15 ms) | 2.0 | 1.5 | 1.5 |
| Longer Pulse (60 ms) | 2.5 | 2.5 | 2.0 |

A longer of activation period may deliver a higher intensity, more complex perfume character and a more substantive perfume presentation than a corresponding perfume using a shorter activation period.

Example 2

Example 2 compares the effect of pulsation times with respect to the number of detectable components by the following method, in situ monitoring of perfume components by GC/MS.

In this method, the testing delivery system is placed in a 2.83 $m^3$ (100 $ft^3$) room with standard room circulation. The samples are collected at (0.2, 3, 6, and 9 feet) 0.06, 0.91, 1.83 and 2.74 m. For each time point a sample is taken at each position. An initial background room sample is taken. The delivery system is placed in the room and turned on. After that, samples are collected at initial, 6, 12 and 18 minutes. The air samples are collected using 4 Gil Air Personal Air Sampler pumps collecting samples for 3 minutes at l L/minute. Samples are collected on 50 mg Tenax TA traps and desorbed using an MPS-2 TDU into a GC/MS system. Samples are analyzed using a 6890/5973 GC/MS with a DB-1 column (1 $\mu$m film thickness, 0.32 mm ID, 60 m length). The data is reported with respect to the number of detectable components as well as Flame Ionization Detector response (FID).

Table 2 shows a higher number of detectable components at all measured distances from the delivery system at the 18 minute sample when longer pulsation time is used. Surprisingly, longer pulsation time produced significantly more detectable components even in the area directly beside the delivery system. Similar trends are observed at the other time measurements.

| Table 2 | Number of Detectable Components Given Distance from the Delivery system | | | |
|---|---|---|---|---|
| | 0.06m(0.2feet) | 0.91 m(3 feet) | 1.83m(6 feet) | 2.74m(9 feet) |
| Short Pulse (10 ms) | 7 | 7 | 6 | 7 |
| Long Pulse (60 ms) | 22 | 17 | 14 | 12 |

Example 3

Example 3 compares the various perforation sizes of the droplet generation element and their effect on deposition and flow rate. To improve perceived intensity of the liquid active material, one may maximize flow rate while minimizing deposition. In this example, the delivery system provides deposition of less than about 6 $\mu$g and flow in the range of about 30 mg/hr and greater.

Flow rates are measured as weight loss over time. The weight of the delivery system is measured using a Mettler Toledo Excellence Xs403S response 0.001 g over time and the weight difference (in mg) was divided by the time period in hours to give a flow rate in mg/hr.

Plume height is determined by placing a delivery system into the center of a cylindrical chamber. At the rear of the chamber, behind the delivery system a darkened screen is provided. A ruler is positioned so that the zero cm mark is directly in line with the top of the delivery system. The vertical distance of the plume reached is measured by the ruler and the average plume height, in cm, is taken from five consecutive sprays.

Deposition of is determined by collecting the non-volatilized or re-deposited liquid active materials on three filter papers, Whatman 40 (Ashless, 18.5 cm Catalog number - 1440 185). Two of filter papers are placed under the bottom edge of the delivery system. The third filter paper is cut to fit the top of the delivery system and placed with the hole cut out over the center of the delivery system. The delivery system is placed in a room and run for a period of 17 to 18 hours. Filter papers are extracted using 5ml of a internal standard solvent solution containing 11.4mg of C14 in 250 ml of methanol. All samples are analyzed by injecting 1 microliter onto a 6890/5973 GC/MS with a DB-1 column (1 $\mu$m film thickness, 0.32 mm ID, 60 m length). Quantitative results are calculated by referencing to a known standard.

Droplet-size distributions are measured using an Oxford Laser Visisizer System. This technique uses high-speed, high-resolution digital photography to acquire images of the spray. The laser light is diffused and used as a backlight (not a beam); the sample volume is approximately 1 mm3. These images are then analyzed by the Visisizer software to yield a droplet size distribution. The measured distributions appear rougher then distributions measured with other techniques (such as laser distraction) since this method actually counts and sizes individual particles. Relevant spray statistics are then calculated from the raw droplet-size data. In most cases, at least 2000 droplets are used to calculate the statistics.

Table 3 illustrates that the a perforation size of about 5 microns to about 7 microns maximizes flow rate and minimizes deposition

Table 3

| Activation period (ms) | Deactivation period (ms) | Perforation Size ($\mu$m) | Droplet size ($\mu$m) | Plume height (cm) | Deposition ($\mu$g) | Flow Rate (mg/hr) |
|---|---|---|---|---|---|---|
| 15 | 10 | 4.5 | 6.5 | 7 | 6 | 10 |
| 45 | 10 | 8 | 11.80 | 10 | X | 42 |
| 60 | 2.5 | 4.5 | 7.40 | 8 | 3 | 35 |
| 60 | 5 | 5.6 | 7.84 | 9 | 4 | 34 |
| 60 | 10 | 7 | 11.00 | 10 | 4 | 32 |
| 60 | 10 | 8 | 11.90 | 11 | 12 | 45 |

(continued)

| Activation period (ms) | Deactivation period (ms) | Perforation Size ($\mu$m) | Droplet size ($\mu$m) | Plume height (cm) | Deposition ($\mu$g) | Flow Rate (mg/hr) |
|---|---|---|---|---|---|---|
| 75 | 10 | 8 | 12.30 | 12.5 | 13 | 47 |
| 100 | 2.5 | 4.5 | 6.70 | 9.5 | 3 | 33 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

While particular embodiments of the present invention have been illustrated and described, it would be obvious to those skilled in the art that various other changes and modifications can be made without departing from the scope of the invention defined by appended claims.

**Claims**

1. A method of distributing a liquid active material to an atmosphere, said method comprising the steps of:

   (a) providing a quantity of said liquid active material:
   (b) providing an electromechanical transducer (3) in communication with said liquid active material;
   (c) activating said electromechanical transducer (3) to distribute said liquid to said atmosphere; and
   (d) carrying out said activation for a first period of time of between 60 and 500 milliseconds

   wherein said electromechanical transducer is coupled or decoupled to a perforated structure having a plurality of perforations (2a, 2b, 2c), said perforations having a diameter of 4 microns to 7 microns.

2. A method according to claim 1 further comprising providing a quantity of a second liquid:

   (a) providing an electromechanical transducer (3) in communication with said second liquid;
   (b) activating said electromechanical transducer (3) to distribute said second liquid to said atmosphere; and carrying out said activation for an alternate period of time of at least 20 milliseconds.

3. A method according to claim 2 wherein the liquid active material is distributed to said atmosphere for a first period of time and said second liquid is distributed to said atmosphere for said alternate period, said first period and said alternate period being different.

**Patentansprüche**

1. Verfahren zum Verteilen eines flüssigen Wirkstoffes in einer Atmosphäre, wobei das Verfahren die folgenden Schritte umfasst:

   (a) Bereitstellen einer Menge des flüssigen Wirkstoffes;
   (b) Bereitstellen eines elektromechanischen Wandlers (3) in Verbindung mit dem flüssigen Wirkstoff;
   (c) Aktivieren des elektromechanischen Wandlers (3), um die Flüssigkeit in der Atmosphäre zu verteilen; und
   (d) Durchführen der Aktivierung für einen ersten Zeitraum zwischen 10 und 500 Millisekunden, wobei der elektromechanische Wandler mit einer perforierten Struktur, die mehrere Perforationen (2a, 2b, 2c) aufweist, gekoppelt oder davon entkoppelt ist, wobei die Perforationen einen Durchmesser von 4 Mikrometer bis 7 Mikrometer aufweisen.

2. Verfahren nach Anspruch 1, ferner umfassend das Bereitstellen einer Menge einer zweiten Flüssigkeit;

   (a) Bereitstellen eines elektromechanischen Wandlers (3) in Verbindung mit der zweiten Flüssigkeit;
   (b) Aktivieren des elektromechanischen Wandlers (3), um die zweite Flüssigkeit in der Atmosphäre zu verteilen; und Durchführen der Aktivierung für einen alternativen Zeitraum von mindestens 20 Millisekunden.

**3.** Verfahren nach Anspruch 2, wobei der flüssige Wirkstoff für einen ersten Zeitraum in der Atmosphäre verteilt wird und die zweite Flüssigkeit für den alternativen Zeitraum in der Atmosphäre verteilt wird, wobei der erste Zeitraum und der alternative Zeitraum unterschiedlich sind.

**Revendications**

**1.** Procédé de distribution d'un matériau actif liquide dans une atmosphère, ledit procédé comprenant les étapes consistant à :

(a) fournir une quantité dudit matériau actif liquide ;
(b) fournir un transducteur électromécanique (3) en communication avec ledit matériau actif liquide ;
(c) activer ledit transducteur électromécanique (3) pour distribuer ledit liquide dans ladite atmosphère ; et
(d) effectuer ladite activation pendant une première période de temps comprise entre 60 et 500 millisecondes, dans lequel ledit transducteur électromécanique est couplé à ou désaccouplé d'une structure perforée possédant une pluralité de perforations (2a, 2b, 2c), lesdites perforations ayant un diamètre compris entre 4 microns et 7 microns.

**2.** Procédé selon la revendication 1
consistant en outre à fournir une quantité d'un second liquide ;

(a) fournir un transducteur électromécanique (3) en communication avec ledit second liquide ;
(b) activer ledit transducteur électromécanique (3) pour distribuer ledit second liquide dans ladite atmosphère ; et effectuer ladite activation pendant une autre période de temps d'au moins 20 millisecondes.

**3.** Procédé selon la revendication 2, dans lequel ledit matériau actif liquide est distribué dans ladite atmosphère pendant une première période de temps et ledit second liquide est distribué dans ladite atmosphère pendant ladite autre période, ladite première période et ladite autre période étant différentes.

Fig. 1

Fig. 2

Fig. 3

Fig. 4

**EP 2 086 314 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3543122 A
- US 3613041 A
- US 4479609 A
- US 4533082 A
- US 4790479 A
- US 5519179 A
- US 5297734 A
- US 6341732 B
- US 6378780 B
- US 6396462 B
- WO 03047766 A
- US 20060175426 A
- US 5518179 A
- WO 02068128 A